# EUROPÄISCHE PATENTANMELDUNG

(11) **EP 4 781 955 A1**
(43) Veröffentlichungstag der Anmeldung: **29.07.2026**
(21) Anmeldenummer: 25153916.9
(22) Anmeldetag: 24.01.2025
(51) Int. Cl.: A61F 7/00, A61F 7/10

(54) **VORRICHTUNG ZUR KÜHLUNG UND/ODER ERWÄRMUNG EINER KÖRPERPARTIE DURCH AUFLAGE AUF DIE HAUT**

(71) Anmelder: Josela GmbH, 73054 Eislingen (DE)
(72) Erfinder: Lehner, Lino, 73066 Uhingen/Nassach (DE); Daub, Semira, 73054 Eislingen/Fils (DE); Hess, Sebastian, 73614 Schorndorf (DE)
(74) Vertreter: Witte, Weller und Partner Patentanwälte mbB Stuttgart

(57) **Zusammenfassung**

Bekannt sind Vorrichtungen (10) zur Kühlung und/oder Erwärmung einer Körperpartie durch Auflage auf die Haut. Solche Vorrichtungen können ein Flüssigkeitsreservoir (80) mit einer formflexiblen Anlagewandung (82) zur Auflage auf der Haut aufweisen. Sie können weiterhin eine Mehrzahl von einzelnen elektrisch betriebenen Kühl- und/oder Heizelementen (50) aufweisen, die an einer der Anlagewandung (82) gegenüberliegenden Gegenwandung (83) derart angebracht sind, dass sie Flüssigkeit im Flüssigkeitsreservoir (80) erwärmen oder abkühlen können.

Es wird vorgeschlagen, die Vorrichtung (10) gegenüberliegend zum Flüssigkeitsreservoir (80) eine Außenschale (20) aufweisen zu lassen, die eine Mehrzahl von gegeneinander beweglichen Schalenelementen (22, 23, 24, 25) umfasst.

## Beschreibung

### ANWENDUNGSGEBIET UND STAND DER TECHNIK

Die Erfindung betrifft eine Vorrichtung zur Kühlung und/oder Erwärmung einer Körperpartie durch Auflage der Vorrichtung auf die Haut.

Gattungsgemäße Vorrichtungen umfassen ein Flüssigkeitsreservoir mit einer formflexiblen Anlagewandung zur Auflage auf der Haut und eine Mehrzahl von einzelnen elektrisch betriebenen Kühl- und/oder Heizelementen, die an einer der Anlagewandung gegenüberliegenden Gegenwandung derart angebracht sind, dass sie Flüssigkeit im Flüssigkeitsreservoir erwärmen oder abkühlen können.

Solche Vorrichtungen werden bestimmungsgemäß genutzt, um auf eine Hautpartie aufgelegt zu werden und diese Hautpartie zu kühlen oderzu erwärmen. Es kann sich beispielsweise um eine Kühlung handeln, die einer Schwellung entgegenwirken soll.

Eine entsprechende Vorrichtung ist beispielsweise aus der DE 102021122642 A1 bekannt.

Als problematisch bei Vorrichtungen der beschriebenen Art hat sich insbesondere die Wärmeabführung erwiesen. Da gattungsgemäße Vorrichtungen an die jeweilige Körperpartie anpassbar sein sollen, müssen sie ein gewisses Maß an Beweglichkeit gestatten. Eine solche Beweglichkeit ist jedoch schwer umzusetzen, ohne dabei die Ventilation zu erschweren. So ist beispielsweise eine Außenseite der Vorrichtung, die durch ein textiles Gewebe gebildet wird, meist zu isolierend und führt zu einem Wärmestau im Gerät. Es wäre bevorzugt, eine starre Außenschale zu verwenden, die Belüftung vereinfacht, doch eine solche starre Außenschale behindert die Relativbeweglichkeit und schied daher in der Vergangenheit aus.

### AUFGABE UND LÖSUNG

Aufgabe der Erfindung ist es, eine gattungsgemäße Vorrichtung zur Kühlung und/oder Erwärmung einer Körperpartie dahingehend weiterzubilden, dass diese robust im Handling ist und einen guten Wärmeaustausch mit der Umgebung ermöglicht.

Vorgeschlagen wird zu diesem Zweck eine Vorrichtung zur Kühlung und/oder Erwärmung einer Körperpartie durch Auflage auf die Haut. Die Vorrichtung, die insbesondere als Gesichtsmaske ausgestaltet sein kann, aber auch für andere Körperpartien verwendet oder spezifisch angepasst sein kann, weist ein Flüssigkeitsreservoir mit einer formflexiblen Anlagewandung zur Auflage auf der Haut auf. Im Flüssigkeitsreservoir, das vorzugsweise als flacher Flüssigkeitsbeutel gestaltet ist, ist im befüllten Zustand eine Flüssigkeit enthalten, bei der es sich im einfachsten Fall um Wasser handeln kann. Diese Flüssigkeit wird von der Vorrichtung erwärmt oder abgekühlt, um die Wärme oder Kälte an die Haut abzugeben und somit dem Wohlbefinden oder auch der Heilung zu dienen.

Die Erwärmung und/oder Abkühlung der Flüssigkeit im Flüssigkeitsreservoir erfolgt über eine Mehrzahl von einzelnen elektrisch betriebenen Kühl- und/oder Heizelementen. Diese sind an einer der Anlagewandung gegenüberliegenden Gegenwandung angebracht und können so die Flüssigkeit erwärmen oderabkühlen. Vorzugsweise umfassen die Kühl- und/oder Heizelemente jeweils Peltierelemente. Insbesondere vorzugsweise weist die Vorrichtung zwischen zwei und zwölf solcher Kühl- und/oder Heizelemente auf, insbesondere vorzugsweise zwischen 3 und 6. Details zu Möglichkeiten der Konfiguration und zur Anbringung der Kühl- und/oder Heizelemente an der Gegenwandung sind im Weiteren noch erläutert.

Die Vorrichtung ist außenseitig durch ei ne Außenschale aus einem starren und formstabilen Material geschützt, insbesondere aus Kunstsoff. Zwischen der Außenschale und derGegenwandungsind verschiedene Komponenten angeordnet, so insbesondere die genannten Kühl- und/oder Heizelemente. Auch eine Steuerelektronik zur Steuerung der Kühl- und/oder Heizelemente kann hier vorgesehen sein. Weiterhin kann hier eine Batterie angeordnet sein, wobei bevorzugt ist, dass die Vorrichtung stattdessen dauerhaft an einer externen Stromquelle angeschlossen ist, beispielsweise über USB an einer sogenannten Powerbank oder an einem Netzteil. Vorzugsweise ist mit Ausnahme dieser externen Stromquelle keine weitere externe Komponente für den Betrieb erforderlich. Stattdessen sind vorzugsweise alle flüssigkeitsführenden Teile sowie die Kühl- und/oder Heizelemente und die Steuerelektronik Teil der einheitlichen Vorrichtung, die somit sehr gut als mobile Vorrichtung verwendbar ist.

Die genannte Außenschale ist erfindungsgemäß als Außenschale ausgebildet, die eine Mehrzahl von gegeneinander beweglichen Schalenelementen umfasst, nicht unähnlich einer Schuppenstruktur. Die Schalenelemente sind in sich starr, aber gegeneinander beweglich.

Das Flüssigkeitsreservoir ist vorzugsweise als flacher Flüssigkeitsbeutel vorgesehen, der zumindest im Bereich der Anlagefläche formflexibel ist und sich an die Oberfläche der Haut somit anpassen kann. Vorzugsweise ist der Flüssigkeitsbeutel beidseitig durch Kunststoffwandungen begrenzt. Es können sowohl an der Anlagewandung als auch an der Gegenwandung weitere Lagen vorgesehen sein, so beispielsweise ein fester oder wechselbarer textiler Bezug auf der Anlagewandung oder eine Isolationsschicht auf der Gegenwandung.

Die Außenschale bildet die dem Flüssigkeitsreservoir und dessen Anlagefläche gegenüberliegende Seite der Vorrichtung und weist daher vorzugsweise in etwa die gleiche Größe auf. Sie ist erfindungsgemäß aus der genannten Mehrzahl von Schalenelementen gebildet, die gegeneinander beweglich sind. Um zu verhindern, dass bei der Relativbewegung Spalten relevanter Größe zwischen den Schalenelementen entstehen, ist vorzugsweise eine Überlappung vorgesehen. Insbesondere kann mindestens ein Schalenelement über eine Zunge verfügen, die ein benachbartes Schalenelement untergreift und bei Anwinkelung der Schalenelemente das Entstehen von Lücken minimiert oder vollständig verhindert.

Zwischen der Außenschale und dem Flüssigkeitsreservoir kann ein umlaufender Abstandshalter vorgesehen sein. Bevorzugt ist allerdings, wenn die Schalenelemente bzw. mindestens ein Schalenelement randseitig direkt mit dem Flüssigkeitsreservoir verbunden ist. Vorzugsweise erfolgt dies über eine Naht oder eine Klebeverbindung. Insbesondere kann vorgesehen sein, dass das Flüssigkeitsreservoir in einem Randbereich um einen Befestigungsrand des Schalenelements umgeschlagen ist oder ein textiles Saumband vorgesehen ist, welches das Flüssigkeitsreservoir und das Schalenelement umgreift und zusammenhält.

Zusätzlich zu dem Hauptkörper der Vorrichtung, umfassend die Außenschale und das Flüssigkeitsreservoir sowie die Kühl- und/oder Heizelemente und vorzugsweise auch deren Steuerelektronik, ist bevorzugterweise eine Befestigungsvorrichtung vorgesehen, mittels derer dieser Hauptkörper am Körper befestigt sein kann. Insbesondere kann diese Befestigungsvorrichtung ein Tragband umfassen, vorzugsweise ein längenveränderliches und/oder elastisches Tragband. Dieses kann dann zur Fixierung der Vorrichtung am Körper verwendet werden, beispielsweise in Art eines am Hinterkopf anliegenden Haltebandes, welches eine als Gesichtsmaske verwendbare Vorrichtung trägt.

Wie schon geschrieben, sind die Schalenelemente vorzugsweise primär aus Kunststoff gefertigt und in sich weitgehend starr. Die Beweglichkeit der Schalenelemente gegeneinander erfolgt also nicht primär unter Ausnutzung von Verformung der Schalenelemente, sondern durch die genannte Relativbewegung als Ganzes. Insbesondere vorzugsweise können die Schalenelemente aus einem thermoplastischen Kunststoff wie PP, PE, PC oderABS gefertigt sein.

Die Schalenelemente gestatten zusammen mit dem formflexiblen Flüssigkeitsreservoir eine Anpassung der Vorrichtung an die jeweilige Hautpartie. Die Vorrichtung kann insbesondere als Ganzes gebogen und geknickt werden, um sich beispielsweise an individuelle Gesichtsformen oder andere Körperteile anpassen zu können. So liegt bspw. im Falle eines Gesichtes die gewünschte Wölbung der Vorrichtung und ihres Flüssigkeitsreservoirs bei einem Radius von etwa 30 cm bis 40 cm, während an einem Arm die Wölbung eher mit einem Radius von etwa 5 cm bis 15 cm ideal ist.

Die Schalenelemente weisen vorzugsweise Durchbrechungen auf, insbesondere schlitzförmige Durchbrechungen, die der Abführung von Kälte oder Wärme dienen. Vorzugsweise sind alle Schalenelemente mit solchen Durchbrechungen versehen.

Im einfachsten Fall sind nur zwei Schalenelemente vorgesehen, die vorzugsweise eine etwa gleiche Größe aufweisen (Größenabweichung kleiner 50 %). Vorzugsweise weist die Außenschale jedoch mehr als zwei gegeneinander bewegliche Schalenelemente auf. Bei einer einfachen Gestaltung bilden die Schalenelemente einen einsträngigen Verbund, also beispielsweise drei, vier oder sechs hintereinander und kettenartig aufeinander folgende Schalenelemente.

Bevorzugt ist allerdings eine Gestaltung, bei der mindestens vier Schalenelemente in einer 2x2-Anordnung vorgesehen sind. Bei einer solchen Anordnung bietet die Vorrichtung verschiedene Schwenkachsen, entlang derer die Vorrichtung einknicken kann, insbesondere mindestens zwei zueinander im Wesentlichen im rechten Winkel (>70°, <110°) stehende Schwenkachsen. Damit wird eine dreidimensionale Verformung möglich, die eine besonders gute Anpassung an eine Körperoberfläche ermöglicht.

Als besonders gut hat sich eine Bauweise mit sechs oder acht Schalenelementen in einer 3x2- oder 4x2-Anordnung herausgestellt.

Grundsätzlich ist es möglich, die Beweglichkeit der Schalenelemente dadurch herzustellen, dass diese an unterschiedlichen Orten mit dem Flüssigkeitsreservoir verbunden sind, sodass sie sich entsprechend der Verformung des Flüssigkeitsreservoirs gegeneinander bewegen. Sie sind in diesem Fall miteinander nur mittelbar über das Flüssigkeitsreservoir verbunden.

Von Vorteil ist jedoch, wenn es zumindest zwischen einigen Schalenelementen auch eine unmittelbare Artikulation gibt. Es wird daher vorgeschlagen, dass mindestens zwei Schalenelemente mittelbar über das Flüssigkeitsreservoir beweglich verbunden sind und zusätzlich unmittelbar über eine Gelenkeinrichtung schwenkbar miteinander verbunden.

Es hat sich gezeigt, dass dieses Zusammenspiel der doppelten Anbindung der Schalenelemente aneinander - mittelbar über das Flüssigkeitsreservoir, unmittelbar über eine Gelenkeinrichtung - in der Praxis vorteilhaft ist. Es wird hiermit eine robuste Konstruktion geschaffen, bei der ein Verkanten der Schalenelemente gegeneinander nicht zu befürchten ist.

Vorzugsweise ist eine Konstruktion gewählt, bei der alle Schalenelemente mittelbar über das Flüssigkeitsreservoir beweglich verbunden sind. Gleichzeitig können alle Schalenelemente mit mindestens einem benachbarten Schalenelement über eine Gelenkverbindung unmittelbar verbunden sein. Als vorteilhaft wird angesehen, dass mindestens zwei benachbarte Schalenelemente lediglich mittelbar über das Flüssigkeitsreservoir beweglich verbunden sind, also nicht mit einem benachbarten Schalenglied in artikulierterVerbindungstehen. Dies ermöglicht es insbesondere, einzelne Schalenelemente ausschließlich mittelbar über das Flüssigkeitsreservoir beweglich zu verbinden und damit die Beweglichkeit zu erhöhen. Dies kann für einzelne Schalenelemente insbesondere dann einen Vorteil darstellen, wenn die Vorrichtung mindestens vier Schalenelemente in einer 2x2- oder sechs oder acht Schalenelemente in einer 3x2- oder 4x2-Anordnung umfasst.

Bei einer Gestaltung, bei der die Schalenelemente nicht einen in nur einer Richtung erstreckten Strang aus Schalenelementen bilden, sondern - wie bei einer 2x2-Anordnung - eine in zwei Dimensionen bewegliche Struktur bilden, ist es von besonderem Vorteil, wenn mindestens ein Schalenelement mit mindestens zwei anderen Schalenelementen unmittelbar über eine Gelenkeinrichtung schwenkbar verbunden ist, wobei die jeweiligen Schwenkachsen zueinander nicht parallel sind. Die mindestens zwei hierdurch verbundenen Schalenelemente sind also durch die Gelenkeinrichtung um verschiedene Schwenkachsen relativ beweglich. Ein viertes Schalenelement, das mit den drei genannten Schalenelementen eine 2x2-Matrix bildet, ist vorzugsweise nur mit einem der drei Schalenelemente oder mit keinem der drei Schalenelemente über die Gelenkeinrichtung verbunden.

Für die Gestaltung der Gelenkeinrichtung sind verschiedene Bauweisen möglich. Beispielsweise könnte eine Gelenkteileinrichtungan einem Schalenelement eine Hülsenstrukturaufweisen, die auf einen Scharnierstift des anderen Schalenelements aufgeschoben ist. Die Hülsenstruktur und der Scharnierstift befinden sich dabei in der Ebene der Außenseite der Schalenelemente oder sind nur um wenige Millimeter nach innen versetzt.

Von Vorteil ist es jedoch, wenn die Gelenkeinrichtung zwischen zwei oder mehr Schalenelementen als Gelenkeinrichtung mit versetzter Schwenkachse gestaltet ist, also mit einer echten und/oder virtuellen Schwenkachse, die gegenüber der Außenseite der Schalenelemente deutlich nach innen und damit in Richtung des Flüssigkeitsreservoirs versetzt ist. Während eines Verschwenkens der Schalenelemente gegeneinander ist die Schwenkachse dabei von einer Außenseite der Schalenelemente um mindestens 10 mm nach innen beabstandet. Die Wirkung dessen ist, dass die Schwenkachse der Gelenkeinrichtung in die Nähe des Flüssigkeitsreservoirs verlagert wird oder idealerweise sogar fast bis in die Ebene des Flüssigkeitsreservoirs gelangt. Dies ist von Vorteil, da dann das Flüssigkeitsreservoir, gegebenenfalls mit Falzlinien versehen, und die Gelenkeinrichtung ähnliche Schwenkachsen definieren und somit ein besonders leichtgängiges Verformen der Vorrichtung für den konkreten Anwendungsfall ermöglicht wird.

Eine besonders vorteilhafte Möglichkeit zur Ausgestaltung einer solchen Gelenkeinrichtung mit nach innen versetzter Schwenkachse besteht darin, an einer Gelenkteileinrichtung eine Kulisseneinrichtung und an der anderen Gelenkteileinrichtung einen Kulissenstift vorzusehen, der in die Kulisse hineinragt. Die Kulisseneinrichtung ist dabei gekrümmt und definiert damit eine nach innen versetzte virtuelle Schwenkachse, ohne dass an dieser Stelle eine tatsächliches Achselement vorgesehen sein muss, was aufgrund des Flüssigkeitsreservoirs kaum realisierbar wäre.

Wenn die Gelenkeinrichtung als 3D-Gelenkeinrichtungzwischen drei Schalenelementen vorgesehen ist, verfügt sie vorzugsweise über mindestens drei Gelenkteileinrichtungen an den drei Schalenelementen, die gemeinsam über zwei gekrümmte Kulissen und zwei in die gekrümmten Kulissen eingreifende und vorzugsweise ebenfalls gekrümmte Kulissenstifte verfügen. Diese Bauweise mit gekrümmten Kulissenstiften ermöglicht den beschriebenen Vorteil der Schaffung einer virtuellen Schwenkachse und gestattet es gleichzeitig durch die Krümmung des Kulissenstifts, dass dieser zusammen mit seinem Schalenelement in einer zusätzlichen Schwenkrichtung eingeschwenkt wird und der Kulissenstift dennoch weiterhin in der zugeordneten Kulisse verbleiben kann.

Insbesondere vorzugsweise umfassen die Schalenelemente mindestens vier Schalenelemente in einer T-Anordnung. Diese T-Anordnung umfasst insbesondere vier von sechs Schalenelementen in einer 2x3-Anordnung. Die vier Schalenelemente sind über eine Gelenkeinrichtung beweglich miteinander verbunden. Insbesondere hat sich eine Bauweise als vorteilhaft herausgestellt, bei der zwei zentrale Schalenelemente zwar unmittelbar benachbart sind, aber nicht direkt durch Gelenkteileinrichtungen miteinander verbunden. Stattdessen sind sie mittelbar miteinander verbunden, da sie jeweils unmittelbar mit zwei außenliegenden Schalenelementen beweglich verbunden sind. Diese Bauweise verringert die Komplexität des Aufbaus und hat sich in der Praxis als vorteilhaft herausgestellt.

Die Ausgestaltung der Kühl- und/oder Heizelemente ist auf verschiedene Weise möglich. Eine bevorzugte Bauweise sieht vor, dass die Kühl- und/oder Heizelemente Peltierelemente zur Erzeugung von Wärme und Kälte in der Flüssigkeit umfassen. Zusätzlich umfassen sie vorzugsweise einen Ventilator, um Wärme auf der dem Flüssigkeitsreservoir abgewandten Seite abzuführen.

Die Kühl- und/oder Heizelemente weisen jeweils eine Kontaktfläche zur Abgabe der Wärme oder Kälte an die Flüssigkeit im Flüssigkeitsreservoir auf. Vorzugsweise liegt diese Kontaktfläche unmittelbar an der Flüssigkeit an. Insbesondere kann dies erzielt werden, indem in der Gegenwandung des Flüssigkeitsreservoirs mindestens eine Aussparung vorgesehen ist, im Bereich dererdie Flüssigkeit unmittelbar an der Kontaktfläche eines Kühl- und/oder Heizelements anliegt. Die Abführung oder Zuführung der Wärme durch die Gegenwandung selbst entfällt hiermit zumindest zum Teil.

Im Falle eines Peltierelements wird die Kontaktfläche vorzugsweise durch eine strukturintegrierte Fläche des Peltierelements gebildet, also eine Fläche, die unmittelbar für die Abgabe oder Aufnahme von Wärme verantwortlich ist und untrennbar mit den thermoelektrischen Modulen verbunden ist und die damit eine direkte Funktionseinheit des Peltierelements darstellt. Weitere Zwischenelemente zwischen dieser strukturintegrierten Fläche des Peltierelements und der Flüssigkeit sind vorzugsweise zu vermeiden.

Abweichend von der vorgeschlagenen Bauweise sind jedoch auch Bauweisen ohne die beschriebenen Aussparungen möglich. Zwar ist in solchen Fällen die Wärmeübertragung der Flüssigkeit zur Umgebung mittels der Kühl- und/oder Heizelemente verschlechtert. Zur Erzielung einer einfacheren Abdichtung des Flüssigkeitsreservoirs kann dies jedoch akzeptabel sein. Denkbar ist auch, zumindest im Bereich der Kühl- und/oder Heizelemente eine Gegenwandung mit verringerter Wandungsstärke zu verwenden.

Vorzugsweise sind die Kühl- und/oder Heizelemente jeweils mittels eines Trägers mit der Gegenwandung verbunden. Ein solcher Träger ist eine einteilige oder mehrteilige Struktur, die fest mit der Gegenwandungverbunden ist und mittelbar ein Kühl- und/oder Heizelement in Position hält. Der Träger kann insbesondere auch für die Abdichtung im Falle einer Gestaltung mit Aussparung in der Gegenwandung geeignet sein.

Insbesondere vorzugsweise weist der Träger eine oder zwei Dichtungen auf. Eine erste Dichtung ist eine umlaufende Dichtung, die an einem die Aussparung umgebenden Randbereich der Gegenwandung anliegt. Diese Dichtung verhindert, dass Flüssigkeit zwischen einem Trägerteil und der Gegenwandung entweichen kann. Eine zweite Dichtung kann dafür vorgesehen sein, an der Kontaktfläche des Kühl- und/oder Heizelements anzuliegen. Sie verhindert hier den Durchtritt von Flüssigkeit. Vorzugsweise sind beide Arten von Dichtungen vorgesehen. Die erste Dichtung kann jedoch beispielsweise verzichtbar sein, wenn der Träger und die Gegenwandung thermisch gefügt und hierdurch dicht sind. Die genannten Dichtungen sind vorzugsweise als einfache O-Ringe ausgebildet.

Der Träger weist im Falle einer Gestaltung mit einer Aussparung in der Gegenwandung vorzugsweise zwei Trägerelemente auf, die auf gegenüberliegenden Seiten der Gegenwandung vorgesehen sind. Vorzugsweise ist eines der Trägerelemente dabei zum überwiegenden Teil im Flüssigkeitsreservoir angeordnet. Die Trägerelemente sind miteinander verbunden und klemmen dabei die Gegenwandung zwischen sich ein. Zusätzlich ist vorzugsweise zur genannten Aussparung in einem die Aussparung umgebenden Randbereich der Gegenwandung mindestens eine Montageaussparungvorgesehen, durch die hindurch die beiden Trägerelemente in Kontakt miteinanderstehen oder aneinander befestigt sind. Die genannten umlaufenden Dichtungen sind vorzugsweise an dem im Flüssigkeitsreservoir angeordneten Trägerelement vorgesehen.

Die Träger können zusätzlich zu ihrer Funktion, die Kühl- und/oder Heizelemente an der Gegenwandung in Position zu halten, die Funktion übernehmen, die Schalenelemente zu tragen. Auch sind Träger möglich, die selbst kein Kühl- und/oder Heizelement tragen, jedoch ein Schalenelement tragen. Vorzugsweise ist mindestens ein Träger vorgesehen, der selbst kein Kühl- und/oder Heizelement trägt, jedoch einen Aufnahmeraum für die genannte Steuerelektronik begrenzt.

Insbesondere vorteilhaft ist es, wenn mindestens ein Schalenelement mindestens ein Bedienelement und/oder mindestens ein Anzeigeelement aufweist. Es ist insbesondere sinnvoll, diese Elemente an dem Schalenelement vorzusehen, das mit einem Träger verbunden ist, der den genannten Aufnahmeraum für die Steuerelektronik begrenzt. Das Anzeigeelement kann in einem Display oder einer LED oder einer Mehrzahl von LEDs bestehen. Das Bedienelement umfasst vorzugsweise mindestens einen An-/Aus-Schalter.

Vorzugsweise ist mindestens ein Schalenelement ortsfest an einem Träger oder an dem mittels des Trägers mit der Gegenwandung verbundenen Kühl- und/oder Heizelement befestigt. Hierdurch wird eine relative Ortsfestigkeit zwischen der Gegenwandung im Bereich eines Kühl- und/oder Heizelements und dem Schalenelement hergestellt, welches das Kühl- und/oder Heizelement überdeckt.

Die relative Ortsfestigkeit zwischen dem Kühl- und/oder Heizelement und derdieses Kühl- und/oder Heizelement überdeckenden Schale ist insbesondere in Hinblick auf die Wärmeabführung von Vorteil. Lüftungsöffnungen oder Lüftungsschlitze können dadurch relativ zum Kühl- und/oder Heizelement optimal platziert werden.

Durch die Verbindung des Schalenelements mit dem Träger ist der Flüssigkeitsbeutel im Bereich seiner Gegenwandung mit den Schalenelementen vergleichsweise starr verbunden. Der Flüssigkeitsbeutel ist in sich verformbar und erfüllt damit eine Art Scharnierfunktion für den Schalenelemente, insbesondere unterstützt durch Siegellinien oder Falzlinien oder durch anderweitige Verbindungen zwischen Anlagewandung und Gegenwandung. Vorzugsweise ist das Flüssigkeitsreservoir als Mehrkammerbeutel ausgebildet, wobei die Kammern voneinander isoliert sind oder einen Flüssigkeitsaustausch zwischen ihnen ermöglichen. Die Grenzen der Kammern gegeneinander bilden vorzugsweise die Falzlinien.

Im Falle eines solchen Mehrkammerbeutels ist es bevorzugt, wenn jedem der gegeneinander beweglichen Schalenelemente eine Kammer zugeordnet ist und der jeweilige Abschnitt der Gegenwandung durch einen Träger mit dem zugeordneten Schalenelement verbunden ist.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 und 2 zeigen eine erfindungsgemäße Kühl- und/oder Heizvorrichtung in zwei Perspektiven.
Fig. 3 zeigt die Kühl- und/oder Heizvorrichtung in einer Schnittdarstellung.
Fig. 4 zeigt die Kühl- und/oder Heizvorrichtung bei ausgeblendetem Flüssigkeitsreservoir.
Fig. 5 zeigt die Außenschale der Kühl- und/oder Heizvorrichtung von der Innenseite.
Fig. 6 zeigt die Kühl- und/oder Heizvorrichtung bei abgenommener Außenschale.
Fig. 7 zeigt die Außenschale der Kühl- und/oder Heizvorrichtung in zerlegter Form.
Fig. 8 bis 10 zeigen ein einzelnes Kühl- und/oder Heizelement einschließlich eines zugeordneten Trägers in verschiedenen Darstellungen.
Fig. 11A und 11B zeigen nochmals die Kühl- und/oder Heizvorrichtung von vorne und von hinten.
Fig. 12A und 12B sowie Fig. 13A und 13B zeigen alternative Bauformen von erfindungsgemäßen Kühl- und/oder Heizvorrichtungen.

### DETAILLIERTE BESCHREIBUNG DER AUSFÜHRUNGSBEISPIELE

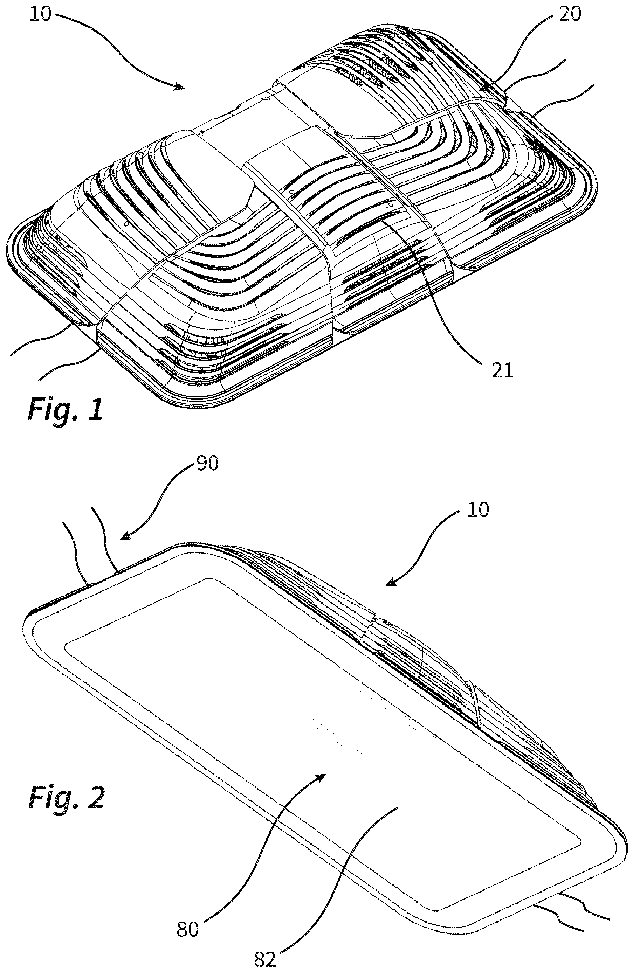

Fig. 1 und 2 zeigen eine erfindungsgemäße Kühl- und/oder Heizvorrichtung 10, die dafür ausgebildet ist, eine Körperpartie zu erwärmen oder zu kühlen.

Die Figuren 1 und 2 zeigen die Vorrichtung 10 und insbesondere deren Hauptkörper in zwei Perspektiven, nämlich in Fig. 1 die Rückseite des Hauptkörpers, die bei bestimmungsgemäßer Verwendung am Körper nach außen weist, und in Fig. 2 die Vorderseite des Hauptkörpers mit einer formflexiblen Anlagewandung 82, die bestimmungsgemäß auf die betreffende Hautpartie, beispielsweise das Gesicht, aufgelegt wird.

Die genannte Anlagewandung 82 stellt gleichzeitig die Außenwandung eines Flüssigkeitsreservoirs 80, das in der Art eines flachen Beutels ausgebildet ist.

Die Rückseite des Hauptkörpers wird durch eine Außenschale 20 gebildet, die aus mehreren Schalenelementen 22, 23, 24, 25 zusammengesetzt ist, wie im Weiteren noch erläutert wird.

An den Außenseiten des Hauptkörpers ist ein elastisches und/oder längenveränderliches Halteband 90 vorgesehen, das beispielsweise im Falle einer Gesichtsmaske um den Hinterkopf gelegt wird.

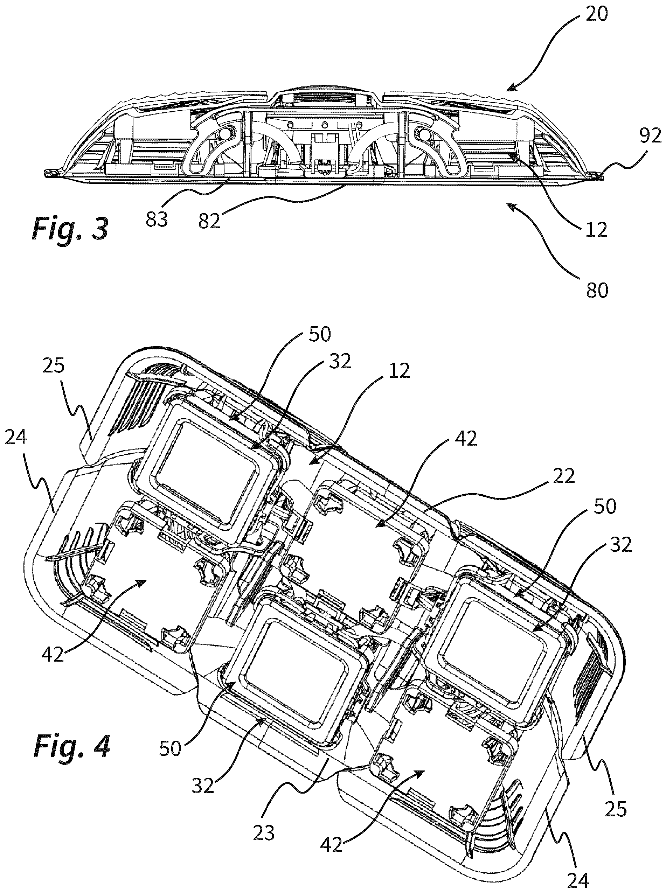

Fig. 3 zeigt die Vorrichtung 10 in einer Schnittdarstellung. Es ist hier zu erkennen, dass das Flüssigkeitsreservoir 80 durch die genannte Anlagewandung 82 und gegenüberliegend hierzu durch eine Gegenwandung 83 begrenzt ist. In einem Randbereich sind diese beiden Wandungen 82, 83 miteinander verbunden. Außenseitig ist ein Saumband 92 vorgesehen, das die Außenschale und den Randbereich des Flüssigkeitsreservoirs 80 umgreift und miteinander verbindet.

Gemeinsam begrenzen das Flüssigkeitsreservoir 80 mit seiner Gegenwandung 83 einerseits und die Außenschale 20 andererseits einen Innenraum 12. In diesem Innenraum 12 sind verschiedene Komponenten der Vorrichtung 10 vorgesehen, die im Weiteren noch erläutert werden. Es handelt sich insbesondere um die Kühl- und/oder Heizelemente 50, die die Flüssigkeit im Flüssigkeitsreservoir 80 erwärmen oder kühlen, sowie um dazugehörende elektrische und elektronische Komponenten, insbesondere eine Steuerelektronik. Weiterhin enthält der Innenraum 12 eine Gelenkeinrichtung, mittels derer die verschiedenen Schalenelemente der Außenschale 20 gegeneinander beweglich geführt werden. Insgesamt sind in diesem Ausführungsbeispiel sechs gegeneinander bewegliche Schalenelemente vorgesehen.

Die Schalenelemente weisen zum Teil dünnwandige Zungen auf, die unter benachbarte Schalenelemente greifen, um das Entstehen großer Spalten in der Außenschale im Zuge einer Bewegung zu verhindern.

Fig. 4 zeigt die Vorrichtung bei ausgeblendetem Flüssigkeitsreservoir 80. Hier ist zu erkennen, dass die sechs Schalenelemente 22, 23, 24, 25 jeweils einen Träger32, 42 aufweisen, der eine Verbindung mit dem Flüssigkeitsreservoir 80 schafft. Die sechs Träger 32, 42 sind auf ihrer in Fig. 4 abgewandten Seite an den sechs Schalenelementen 22, 23, 24, 25 befestigt. Ihre jeweilige in Fig. 4 nach vorne weisende Seite ist zur Befestigung an der Gegenwandung des in Fig. ausgeblendeten Flüssigkeitsreservoirs 80 vorgesehen.

Wie ersichtlich ist, gibt es zwei grundsätzlich unterschiedliche Arten von Trägern 32, 42. Die drei Träger 42 weisen eine plane Grundfläche auf, die beispielsweise mittels einer Klebe- oder Schweißverbindung am Flüssigkeitsreservoir 80 befestigt werden kann. Die drei Träger 32 stellen nicht nur eine Verbindung zwischen der Gegenwandung 83 und dem jeweiligen Schalenelement dar, sondern tragen zusätzlich drei Kühl- und/oder Heizelemente 50. Hier ist vorgesehen, dass das in Fig. 4 ausgeblendete Flüssigkeitsreservoir für die drei Kühl- und/oder Heizelemente 50 jeweils eine Aussparung aufweist und dass die Träger 32 jeweils mehrteilig ausgebildet sind, um im Bereich dieser Aussparung beidseitig der Gegenwandung 83 angeordnete Teile aufzuweisen, die miteinander zur Herstellung einer Klemmverbindung verbunden sind. Auch dies wird im Weiteren noch erläutert.

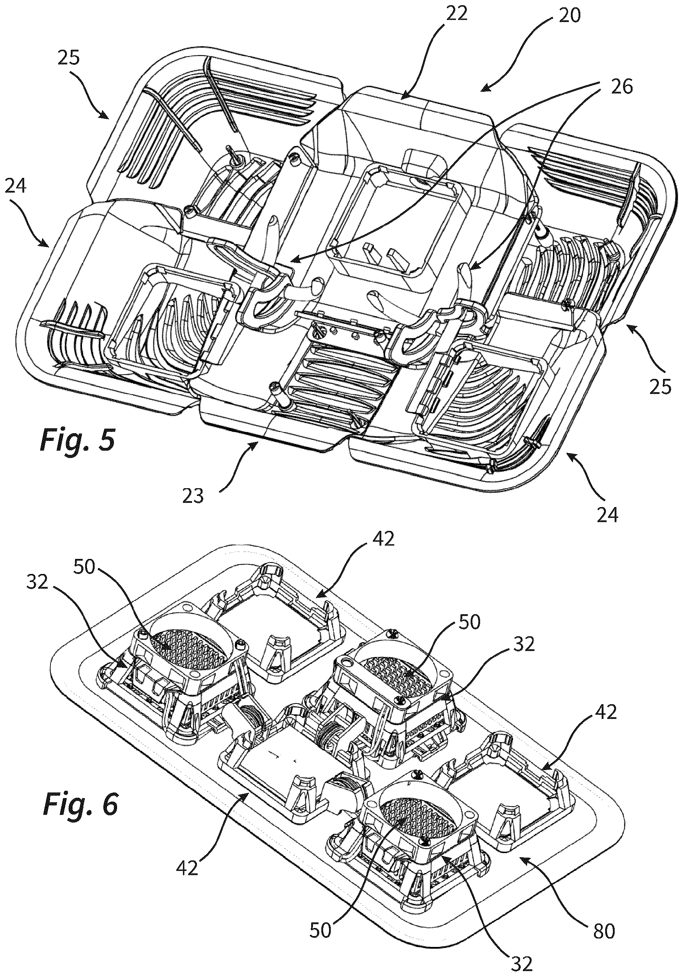

In Fig. 5 lässt sich die isolierte Außenschale 20 und ihre sechs Schalenelemente 22, 23, 24, 25 genauer erkennen. Die Vorrichtung ist im Wesentlichen spiegelbildlich ausgebildet. Von den Schalenelementen 24 und 25 sind jeweils zwei einander entsprechende Schalenelemente 24, 25 vorgesehen.

Wie gut zu erkennen ist, bilden die Schalenelemente 22, 23, 24, 25 eine 3x2-Matrix. Das Schalenelement 22 verfügt über eine Durchbrechung 22C, durch die Strom zugeführt werden kann, insbesondere zu einer in den Figuren nicht näher dargestellten Steuerelektronik über einen USB-C-Anschluss. In Fig. 5 ist am Schalenelement 22 eine innenseitige Wandungsstruktur vorgesehen, die dem Zweck dient, diese Steuerelektronik zu schützen. Alle Schalenelemente sind mit Lüftungsschlitzen ausgestattet.

An das Schalenelement 22 schließt sich in Fig. 5 unterhalb ein Schalenelement 23 an. Außenseitig davon sind die Schalenelemente 24 vorgesehen. Die Schalenelemente 22, 23, 24 sind miteinander über eine Gelenkeinrichtung 26 beweglich verbunden und bilden eine T-förmige Struktur. Die Schalenelemente 25 sind nicht Teil dieserT-förmigen Struktur, da sie anders als die Schalenelemente 22, 23, 24 nicht direkt mit benachbarten Schalenelementen verbunden sind.

Im Falle der T-förmigen Struktur aus den Schalenelementen 22, 23, 24 ist die genannte Gelenkeinrichtung 26 dafür vorgesehen, zwischen den Schalenelementen eine direkte Verbindungzu schaffen, dabei aber die Beweglichkeit der Schalenelemente gegeneinander zu bewahren.

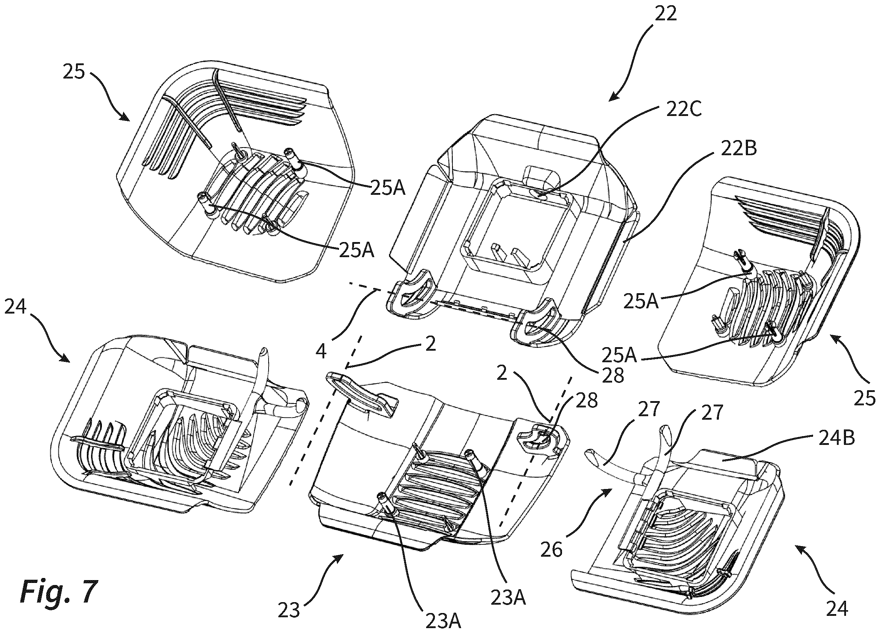

Unter Bezugnahme auf Fig. 7 wird dies näher erläutert. Die Gelenkeinrichtung26 umfasst im Wesentlichen die folgenden Komponenten: An den Schalenelementen 22 sowie 23 sind jeweils Flügel mit gekrümmten Kulissenbahnen 28 vorgesehen. An den Schalenelementen 24 sind korrespondierend hierzu jeweils zwei Kulissenstifte 27 vorgesehen, die im rechten Winkel zueinander stehen. Die Kulissenstifte selbst sind ebenfalls gekrümmt.

Der Vergleich der Fig. 7 und 5 zeigt das Zusammenwirken: Die jeweils zwei gekrümmten Kulissenstifte 27 der beiden Schalenelemente 25 sind in die Kulissenbahnen 28 der Schalenelemente 22 und 23 eingefügt. Sie verbinden dadurch die Schalenelemente 22 und 23 mittelbar und stellen unmittelbar eine Relativbeweglichkeit zwischen den Schalenelementen 25 einerseits und den Schalenelementen 22, 23 andererseits her.

Die Verwendung von gekrümmten Kulissenbahnen 28 und darin geführten, ebenfalls gekrümmten Kulissenstiften führt dazu, dass die in der genannten T-Form angeordneten Schalenelemente 22, 23, 24 dreidimensional gegeneinander schwenkbeweglich sind. Dadurch wird die dreidimensionale Anpassbarkeit der Vorrichtung 10 erzielt. Bezogen auf Fig. 7 können die Schalenelemente 22, 23 um die Achse 4 gegeneinander verschwenkt werden, während die Schalenelemente 24 um die Achse 2 gegenüber dem Schalenelement 23 verschwenkbar sind.

Wenngleich die Schwenkbewegung unmittelbar um die genannten Achsen 2 und 4 stattfindet, die durch die Kulissenstifte 27 definiert sind, wird durch die gekrümmte Kulissenbahn jeweils eine hiervon abweichende virtuelle Schwenkachse definiert. Dies ist in Fig. 7 gutzu erkennen: Die Krümmung der Kulissenbahnen 28 ist so gewählt, dass ein den Verlauf vorgebender Mittelpunkt in etwa im Bereich des Flüssigkeitsreservoirs 80 angeordnet ist. Diese Lage der virtuellen Schwenkachse ist vorteilhaft, da die Schwenkbewegung gleichsam doppelt geführt ist, nämlich einerseits durch die Gelenkeinrichtung 26 und deren virtuelle Schwenkachse und andererseits durch das in Art eines Filmscharniers wirkende Flüssigkeitsreservoir 80.

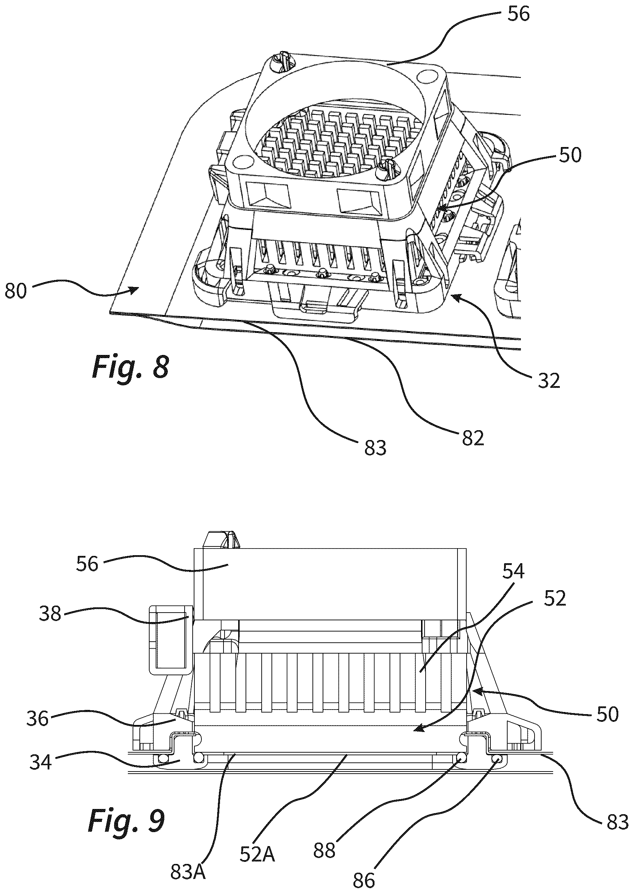

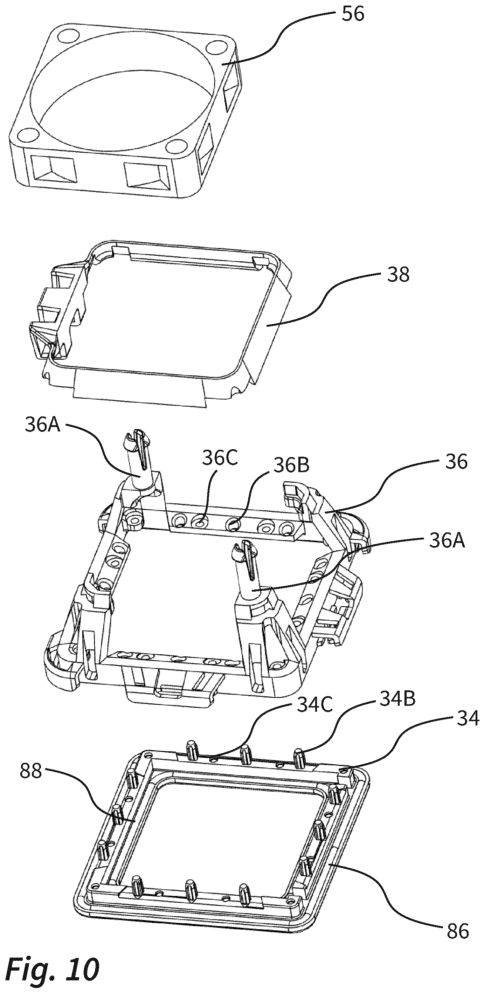

Die Fig. 8 bis 10 verdeutlichen den Aufbau der Kühl- und/oder Heizelemente 50 und derzugehörigen Träger 32. Bezugnehmend zunächst auf Fig. 9 und 10 ist zu erkennen, dass der Träger 32 aus mehreren Trägerelementen 34, 36, 38 zusammengesetzt ist. Das Trägerelement 34 ist, bezogen auf die Explosionsdarstellung der Fig. 10, das unterste Trägerelement. Es ist zum überwiegenden Teil im Flüssigkeitsreservoir 80 angeordnet und verfügt über Ausrichtungs- und/oder Kopplungsstrukturen 34B, die mit einem zweiten Trägerelement 36 auf der gegenüberliegenden Seite der Gegenwandung 83 und dortigen Strukturen 36B zusammenwirken.

Die beiden Trägerelemente 34, 36 weisen weiterhin auch zueinander fluchtend ausgerichtete Schraublöcher 34C, 36C auf, mittels derer die beiden Trägerelemente 34, 36 miteinander gekoppelt werden, sodass sie die Gegenwandung 83 zwischen sich einklemmen. Zusätzlich ist noch ein weiteres Trägerelement 38 vorgesehen.

Wie sich aus Fig. 8 und 9 ersehen lässt, trägt der Träger ein Kühl- und/oder Heizelement 50, das seinerseits ein Peltierelement52 sowie einen Rippenkühler 54 und einen Ventilator 56 umfasst.

Am Trägerelement 34 sind zwei Dichtungen 86, 88 vorgesehen. Die Dichtung 86 liegt im montierten Zustand an der Innenseite der Gegenwandung 83 an und dichtet diese ab. Flüssigkeit kann somit nicht zwischen der Gegenwandung und dem Träger 32 entweichen. Die zweite Dichtung 88 liegt an der strukturintegrierten Kontaktfläche 52A des Kühlelements 50 an und verhindert einen Austritt von Flüssigkeit am Rand der Kontaktfläche 52A.

Die Kontaktfläche 52A des Peltierelements 52 ist durch diesen Aufbau umlaufend abgedichtet und Flüssigkeit aus dem Flüssigkeitsreservoir 80 kann nicht nach außen entweichen. Gleichzeitig steht die Kontaktfläche 52A aufgrund der Aussparungen 83A in der Gegenwandung 83 unmittelbar in Flüssigkeitskontakt, sodass ein sehr effizientes und schnelles Erwärmen und/oder Abkühlen der Flüssigkeit im Flüssigkeitsreservoir 80 erzielt wird.

Wie sich aus den Fig. 8 und 10 ersehen lässt, hat der Ventilator an vier Ecken Befestigungsbohrungen 56A. In Fig. 8 ist dargestellt, dass zwei dieser Befestigungsbohrungen 56A genutzt werden, um mit Befestigungsstiften 36D des Trägerelements 36 zu verrasten.

Die anderen beiden Befestigungsbohrungen 56A werden zur Anbringung der Schalenelemente 23 und 25 genutzt. Wie sich aus Fig. 7 ersehen lässt, weisen diese Schalenelemente jeweils Raststifte 23A, 25A auf, die in die Befestigungsbohrungen 56A eingeschoben und dort verrastet werden können. Hierdurch wird erreicht, dass die Träger 32 und mit ihnen die Kühl- und/oder Heizelemente 50 ortsfest zu den Schalenelementen 23, 25 verrastet werden.

Im Falle der anderen Schalenelemente 22, 24 sind exemplarisch keine Kühl- und/oder Heizelemente 50 unmittelbar zugeordnet. Diese Schalenelemente 22, 24 weisen daher etwas andere Kopplungsstrukturen auf, die bestimmungsgemäß mit den Trägern 42 verrastet werden.

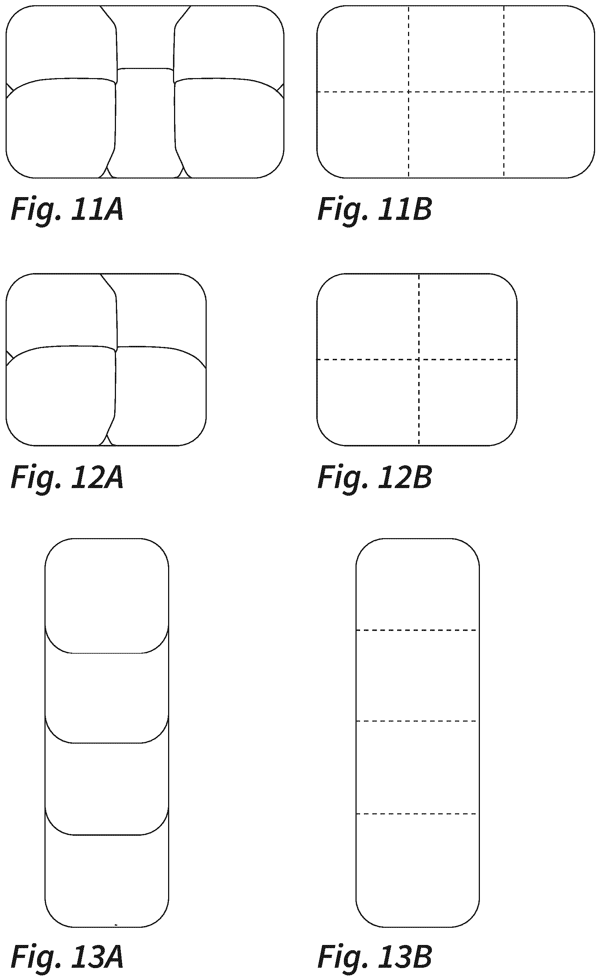

Das bis hier beschriebene Ausführungsbeispiel weist Schalenelemente in einer 3x2-Matrix auf. Die Fig. 11A und 11B verdeutlichen dies nochmals. Fig. 11A zeigt die Konfiguration der Schalenelemente der Außenschale 20. Fig. 11B zeigt, wie korrespondierend hierzu das Flüssigkeitsreservoir 80 in sechs Teile untergliedert ist, die, angebunden durch die Träger 32, 42, jeweils im Wesentlichen ortsfest zu den Schalenelementen verbleiben.

Fig. 12A und 12B zeigen eine alternative Gestaltung mit nur vier Schalenelementen, die gemeinsam die Außenschale bilden. Entsprechend ist auch das Flüssigkeitsreservoir in Fig. 12B in vier Teile untergliedert, die jeweils zu einem Schalenelement im Wesentlichen ortsfest bleiben und gemeinsam mit den Schalenelementen in eine geknickte Konfiguration gebracht werden können.

Fig. 13A und 13B zeigen eine besondere Gestaltung, da hier eine 1x4-Matrix gezeigt ist, also eine Gestaltung, bei der der Flüssigkeitsreservoir gemäß Fig. 13B und die Außenschale gemäß Fig. 13A aus kettenartig aneinander anschließenden Segmenten des Flüssigkeitsreservoirs und Schalenelementen gebildet werden.

## Patentansprüche

**1.** Vorrichtung (10) zur Kühlung und/oder Erwärmung einer Körperpartie durch Auflage auf die Haut mit den folgenden Merkmalen:
a. die Vorrichtung (10) weist ein Flüssigkeitsreservoir (80) mit einer formflexiblen Anlagewandung (82) zur Auflage auf der Haut auf, und
b. die Vorrichtung (10) weist eine Mehrzahl von einzelnen elektrisch betriebenen Kühl- und/oder Heizelementen (50) auf, die an einer der Anlagewandung (82) gegenüberliegenden Gegenwandung (83) derart angebracht sind, dass sie Flüssigkeit im Flüssigkeitsreservoir (80) erwärmen oder abkühlen können, und
c. die Vorrichtung (10) weist gegenüberliegend zum Flüssigkeitsreservoir (80) eine Außenschale (20) auf, die eine Mehrzahl von gegeneinander beweglichen Schalenelementen (22, 23, 24, 25) umfasst.

**2.** Vorrichtung nach Anspruch 1 mit dem folgenden weiteren Merkmal:
a. die Außenschale (20) weist mindestens vier Schalenelemente (22, 23, 24, 25) auf, die in einer 2x2-Anordnung angeordnet sind,
vorzugsweise mit dem folgenden zusätzlichen Merkmal:
b. die Außenschale (20) weist mindestens sechs Schalenelemente (22, 23, 24, 25) auf, die in einer 3x2-Anordnung angeordnet sind.

**3.** Vorrichtung (10) nach Anspruch 1 oder 2 mit dem folgenden weiteren Merkmal:
a. mindestens zwei Schalenelemente (22, 23, 24) sind mittelbar über das Flüssigkeitsreservoir miteinander beweglich verbunden und zusätzlich unmittelbar über eine Gelenkeinrichtung (26) schwenkbar miteinander verbunden,
vorzugsweise mit mindestens einem der folgenden zusätzlichen Merkmale:
b. alle Schalenelemente (22, 23, 24) sind miteinander mittelbar überdas Flüssigkeitsreservoir beweglich verbunden, und/oder
c. mindestens zwei benachbarte Schalenelemente (22, 24; 25) sind miteinander lediglich mittelbar über das Flüssigkeitsreservoir (80) beweglich verbunden.

**4.** Vorrichtung (10) nach Anspruch 3 mit dem folgenden weiteren Merkmal:
a. mindestens ein Schalenelement (24) ist mit mindestens zwei anderen Schalenelementen (22, 23) unmittelbar über eine Gelenkeinrichtung (26) schwenkbar verbunden, wobei die jeweiligen Schwenkachsen (2, 4) zueinander nicht-parallel sind.

**5.** Vorrichtung (10) nach einem der Ansprüche 3 oder4 mit dem folgenden weiteren Merkmal:
a. die Gelenkeinrichtung (26) zwischen zwei oder mehr Schalenelementen (22, 23, 24) ist als Gelenkeinrichtung mit versetzter Schwenkachse (2, 4) gestaltet, wobei die Schwenkachse (2, 4) während eines Verschwenkens der Schalenelemente (22, 23, 24) gegeneinander zumindest phasenweise von einer Außenseite der Schalenelemente (22, 23, 24) um mindestens 10 mm beabstandet ist.

**6.** Vorrichtung (10) nach einem der Ansprüche 2 bis 5 mit den folgenden weiteren Merkmalen:
a. die Gelenkeinrichtung (26) zwischen zwei Schalenelementen ist als Kulissengelenkeinrichtung ausgebildet und verfügt über mindestens zwei Gelenkteileinrichtungen an den zwei Schalenelementen (22, 23, 24), und
b. eine erste Gelenkteileinrichtung weist eine Kulissenbahn (28) auf, vorzugsweise eine gekrümmte Kulissenbahn (28), und
c. eine zweite Gelenkteileinrichtung weist einen Kulissenstift (27) auf, der in der Kulissenbahn (28) der ersten Gelenkteileinrichtung beweglich geführt ist, wobei der Kulissenstift (27) vorzugsweise als gekrümmter Kulissenstift (27) ausgebildet ist.

**8.** Vorrichtung (10) nach einem der Ansprüche 4 bis 7 mit dem weiteren zusätzlichen Merkmal:
a. die Gelenkeinrichtung (26) zwischen drei Schalenelementen (22, 23, 24) ist als 3D-Kulissengelenkeinrichtung ausgebildet und verfügt über mindestens drei Gelenkteileinrichtungen an den drei Schalenelementen (22, 23, 24), die gemeinsam überzwei gekrümmte Kulissenbahnen (28) und zwei in die gekrümmten Kulissenbahnen (28) eingreifende gekrümmte Kulissenstifte (27) verfügt,
vorzugsweise mit dem folgenden zusätzlichen Merkmal:
b. die Schalenelemente umfassen mindestens vier Schalenelemente (22, 23, 24) in einer T-Anordnung, wobei zwei zentrale Schalenelemente (22, 23) mittelbar über zwei außenliegende Schalenelemente (24) gegeneinander beweglich verbunden sind.

**9.** Vorrichtung nach einem der vorstehenden Ansprüche mit den folgenden weiteren Merkmalen:
a. die Kühl- und/oder Heizelemente (50) weisen jeweils eine Kontaktfläche (52A) zur Abgabe der Wärme oder Kälte an die Flüssigkeit im Flüssigkeitsreservoir (80) auf, und
b. in der Gegenwandung (83) des Flüssigkeitsreservoirs (80) ist je Kühl- und/oder Heizelemente mindestens eine Aussparung (83A) vorgesehen, im Bereich derer die Flüssigkeit unmittelbar an der Kontaktfläche (52A) anliegt,
vorzugsweise mit dem folgenden zusätzlichen Merkmal:
c. die Kühl- und/oder Heizelemente (50) weisen Peltierelemente (52) auf und die Kontaktfläche (52A) wird durch eine strukturintegrierte Fläche des jeweiligen Peltierelements (52) gebildet.

**10.** Vorrichtung nach einem der vorstehenden Ansprüche mit dem folgenden weiteren Merkmal:
a. die Kühl- und/oder Heizelemente (50) sind jeweils mittels eines Trägers (32) mit der Gegenwandung (83) verbunden,
vorzugsweise mit dem folgenden weiteren Merkmal:
b. der Träger (32) weist mindestens eine umlaufende Dichtung (86) auf, die an einem die Aussparung (83A) umgebenden Randbereich der Gegenwandung (83) anliegt.

**11.** Vorrichtung nach Anspruch 10 mit dem folgenden weiteren Merkmal:
a. der Träger (32) weist mindestens eine umlaufende Dichtung (88) auf, die an der Kontaktfläche (52A) des zugeordneten Kühl- und/oder Heizelements (50) anliegt.

**12.** Vorrichtung nach Anspruch 10 mit dem folgenden weiteren Merkmal:
a. der Träger (32) weist zwei Trägerelemente (34, 36) auf, die auf gegenüberliegenden Seiten der Gegenwandung (83) vorgesehen sind und unmittelbar miteinander verbunden sind, wobei eines der Trägerelemente (34) zum überwiegenden Teil im Flüssigkeitsreservoir (80) angeordnet ist,
vorzugsweise mit mindestens einem der folgenden zusätzlichen Merkmale:
b. in einem die Aussparung (83A) umgebenden Randbereich der Gegenwandung (83) ist mindestens eine Montageaussparung vorgesehen, durch die hindurch die beiden Trägerelemente (34, 36) in Kontakt miteinander stehen oder aneinander befestigt sind, und/oder
c. mindestens eine der umlaufenden Dichtungen (86, 88) ist an dem im Flüssigkeitsreservoir (80) angeordneten Trägerelement (34) vorgesehen.

**13.** Vorrichtung nach Anspruch 10 oder 11 mit dem folgenden weiteren Merkmal:
a. mindestens ein Schalenelement (22, 23, 24, 25) ist ortsfest an einem Träger (32) oder an dem mittels des Trägers (32) mit der Gegenwandung (83) verbundenen Kühl- und/oder Heizelements (50) befestigt.

**14.** Vorrichtung nach einem der Ansprüche 10 bis 12 mit dem folgenden weiteren Merkmal:
a. es ist mindestens ein Träger (42) an der Gegenwandung (83) angebracht, der nicht der Befestigung eines Kühl- und/oder Heizelements (50) dient und mittels dessen mindestens ein Schalenelement (25) mit dem Flüssigkeitsreservoir (80) verbunden ist.

**15.** Vorrichtung nach einem der vorstehenden Ansprüche mit mindestens einem der folgenden weiteren Merkmale:
a. einige der Schalenelemente (22, 23, 24, 25), vorzugsweise alle Schalenelement, sind mit Belüftungsaussparungen (21) versehen, und/oder
b. mindestens ein Schalenelement (22, 24) verfügt über eine dünnwandige Zunge (22B, 24B), die ein benachbartes Schalenelement (25) untergreift und bei Anwinkelung der Schalenelemente (22, 24; 25) einen offenen Spalt zwischen den Schalenelementen (22, 24; 25) verringert oder verhindert, und/oder
c. die Schalenelemente (22, 23, 24, 25) sind aus Kunststoff gefertigt, insbesondere aus einem thermoplastischen Kunststoff, und/oder
d. die Vorrichtung (10) verfügt über eine Steuerelektronik, wobei die Steuerelektronik vorzugsweise ortsfest zu einem Schalenelement (22) angeordnet ist, dem kein Kühl- und/oder Heizelement (50) zugeordnet ist, und/oder
e. die Vorrichtung (10) weist eine Anschlussbuchse zur Ankopplung eines Stromversorgungskabels auf, wobei die Anschlussbuchse vorzugsweise ortsfest zu einem Schalenelement (25) vorgesehen ist und insbesondere vorzugsweise hinter einer Durchbrechung (22C) in diesem Schalenelement angeordnet ist, und/oder
f das Flüssigkeitsreservoir (80) ist als Mehrkammerbeutel ausgebildet, wobei die Kammern voneinander isoliert sind oder einen Flüssigkeitsaustausch ermöglichen und wobei jedem der gegeneinander beweglichen Schalenelemente (22, 23, 24, 25) eine Kammer zugeordnet ist, wobei die Kammern vorzugsweise durch Siegellinien gebildet werden, im Bereich derer die Anlagewandung und die Gegenwandung in Kontakt miteinander stehen und die Knicklinien bei der Verformung des Flüssigkeitsreservoirs (80) darstellen, und/oder
g. mindestens ein Schalenelement (22, 23, 24, 25) ist randseitig mit dem Flüssigkeitsreservoir (80) verbunden, wobei dies vorzugsweise über eine Naht oder eine Klebeverbindung erfolgt und wobei insbesondere vorzugsweise das Flüssigkeitsreservoir in einem Randbereich um einen Befestigungsrand des Schalenelements (22, 23, 24, 25) umgeschlagen ist oder ein textiles Saumband vorgesehen ist, welches das Flüssigkeitsreservoir (80) und das Schalenelement (22, 23, 24, 25) umgreift, und/oder
h. an mindestens einem Schalenelement (22) ist mindestens ein Bedienelement und/oder mindestens ein Anzeigeelement vorgesehen, und/oder
i. die Vorrichtung (10) verfügt über ein Tragband, vorzugsweise ein längenveränderliches Tragband.
